# EUROPEAN PATENT APPLICATION

(11) **EP 2 374 825 A1**
(43) Date of publication of application: **12.10.2011**
(21) Application number: 09833530.0
(22) Date of filing: 17.12.2009
(51) Int. Cl.: C08F 220/26, A61K 9/127, A61K 31/409, A61K 39/395, A61K 45/00, A61K 47/48, A61P 35/00

(54) **PHOTODYNAMIC THERAPEUTIC AGENT SHOWING ACCUMULATION OF CELL-SPECIFIC FUNCTIONS**

(30) Priority: 17.12.2008 JP 2008321500
(71) Applicant: Keio University, Tokyo 108-8345 (JP); The University of Tokyo, Bunkyo-Ku Tokyo 113-8654 (JP)
(72) Inventor: UEDA, Masakazu, Tokyo 160-8582 (JP); ARAI, Tsunenori, Yokohama-shi Kanagawa 223-8522 (JP); MATSUDA, Sachiko, Tokyo 160-8582 (JP); ISHIHARA, Kazuhiko, Tokyo 113-8654 (JP)
(74) Representative: Denison, Christopher Marcus
(86) International application number: PCT/JP2009/071517
(87) International publication number: WO 2010/071230

(57) **Abstract**

The present invention relates to a cancer-cell-specific polymer compound containing a photosensitizer for photodynamic therapy and having a surface to which an antibody against a cancer-cell-specific antigen binds. An object of the present invention is to provide a photodynamic therapeutic agent that can reach the cancer tissue at a low concentration via active targeting and makes complications (e.g., sunlight hypersensitivity) associated with photodynamic therapy unlikely to occur.

## Description

### Technical Field

The present invention relates to photodynamic therapy and further relates to a photodynamic therapeutic agent comprising a copolymer that: contains a photosensitizer; has an antibody against a cell-specific antigen on its surface; and has a constitutional unit based on 2-methacryloyloxyethyl phosphorylcholine (hereinafter, abbreviated as MPC) and a constitutional unit based on a hydrophobic monomer.

### Background Art

Photodynamic therapy (PDT) is a less-invasive therapy established by taking functional preservation into consideration and using a photosensitizer that is accumulated in cancer and a photochemical reaction due to laser beam irradiation. PDT can be safely and effectively performed. Also, PDT is advantageous in that it can be repeatedly performed, unlike radiation therapy and the like, and it has no toxicity, unlike that of anticancer agents. PDT is expected to serve as a new minimally invasive therapy.

Regarding its molecular aspect, when a cancerous area is irradiated with laser beam, the accumulated tumor-seeking photosensitizer is converted from a singlet state (that is, a ground state) to a triplet state (that is, an excited state) because of increased intramolecular energy level. When the photosensitizer reaches this state, it is photoactivated, resulting in direct generation of active free radicals, or the conversion of triplet oxygen (³O₂) in a ground state to highly active singlet oxygen (¹O₂) as a result of indirect intramolecular energy conversion. These two photoactivation reactions take place simultaneously, and the thus generated products both exhibit the effect of directly damaging cells.

Two drugs, Photofrin and Laserphyrin, have been approved as photodynamic therapeutic agents in Japan. It is known that both drugs are incorporated into tumor tissue at a level about 4 times higher than that in the case of normal tissue. When Photofrin is used, laser irradiation is performed about 48 hours after administration. When Laserphyrin is used, laser irradiation is performed about 4 hours after administration. The drug is eliminated earlier in the case of normal tissue than in the case of cancer tissue, and thus the tumor can be selectively treated in accordance with the time interval. PDT using Photofrin is applied to minimally invasive intrabronchial non-small cell lung cancer partially or completely obstructed esophageal cancer, other types of lung tumor, mesothelioma, early esophageal cancer of Barrett's esophagus, skin cancer, mammary cancer, colorectal cancer, malignant gynecologic tumor, and the like. However, Photofrin and Laserphyrin cause sunlight hypersensitivity as a complication, and thus light shielding is required. Specifically, direct sunlight should be avoided for about 2 to 3 weeks after administration of Photofrin and for about 1 week after administration of Laserphyrin, for example.

Also, regarding *in vivo* components, water has an absorption peak at around 200 nm, which is a longer wavelength than a wavelength of around 700 nm. Oxygenated hemoglobin has bimodal absorption peaks at around 400 nm and 600 nm. An absorption wavelength in the vicinity of 650 nm to 700 nm has the least impact on living- body-derived tissue. To treat even deeper lesions, the development of a photosensitizer having an absorption edge in a long wavelength region has been desired. Photofrin and Laserphyrin have absorption peaks at 630 nm and 664 nm, respectively. Meanwhile, verteporfin has an absorption peak at 690 nm and thus can be said to be more appropriate for the purpose (see patent documents 1 to 3). Verteporfin is also a photosensitizer and is used under the trade name of Visudyne (registered trademark)

(NOVARTIS) for treatment of age-related macular degeneration, but the application thereof for cancer has not been currently approved. However, low-to-moderate grade complement activity of Visudyne was observed at a concentration of 10 µg/ml in an *in vitro* test using human blood, and significant complement activation was observed at a concentration of 100 µg/ml or higher. However, mass administration of Visudyne cannot be performed since the risk of the expression of an anaphylactic reaction due to complement activation cannot be eliminated.

Regarding photodynamic therapy, it has been reported that a photosensitizer for a photodynamic therapeutic agent can be encapsulated in a liposome, following which the liposome is administered (see patent documents 4 and 5). However, it has not been reported to date that such liposome preparations have actually been administered to living organisms via intravenous administration, that the liposome preparations have reached cancer tissue in a cell-specific manner via active targeting, and thus that photodynamic therapy for cancer has succeeded.

Meanwhile, as a polymer with high biocompatibility, a water soluble polymer that is a copolymer having a constitutional unit based on MPC and a constitutional unit based on a hydrophobic monomer has been developed (see patent documents 6 and 7). More than 10 years have already passed since the invention of such polymer. Such polymer is broadly used as a constitutive medical material for medical materials (e.g., artificial pancreas, artificial kidney, or artificial heart), and the safety, stability, and the like of such polymers have been proved. The polymer has a hydrophobic unit and a hydrophilic unit.
Patent documents 1 JP Patent Publication (Kohyo) No. 2006-507307 A
Patent documents 2 JP Patent Publication (Kohyo) No. 2007-522137 A
Patent documents 3 JP Patent Publication (Kohyo) No. 2008-501727 A
Patent documents 4 JP Patent Publication (Kokai) No. 2006-56807 A
Patent documents 5 JP Patent Publication (Kokai) No. 2007-277218 A
Patent documents 6 JP Patent Publication (Kokai) No. 2003-137816 A
Patent documents 7 JP Patent Publication (Kokai) No. 2004-175752 A

### Disclosure of the Invention

An object of the present invention is to provide a photodynamic therapeutic agent that can reach cancer tissue at a low concentration via active targeting, causing fewer complications such as sunlight hypersensitivity that are associated with photodynamic therapy.

The present inventors have intensively examined a method for reducing the onset of sunlight hypersensitivity, which is a complication caused by photodynamic therapy (PDT) and the expression of an anaphylactic reaction, which is a complication caused by the use of verteporfin as a photosensitizer. As a result, the present inventors have attempted to use a polymer (that is, a copolymer) for photodynamic therapy. Such polymer has a constitutional unit based on MPC and a constitutional unit based on a hydrophobic monomer, encapsulates a photosensitizer, and has a surface to which an antibody against an antigen (to be expressed in a cancer-cell-specific manner) binds.

Specifically, a polymer composed of hydrophilic MPC, hydrophobic n-butyl methacrylate (MBA), and p-nitrophenyloxycarbonyl poly(ethylene glycol) methacrylate (MEONP) having an active ester was used. In the present invention, the polymer is referred to as PMBN (poly[2-methacryloyloxyethyl phosphorylcholine (MPC)-co-n-butylmethacrylate (BMA)-co- p-nitrophenyloxycarbonyl poly(ethylene glycol) methacrylate (MEONP)]) or an MPC polymer. The polymer has an active ester that allows it to bind to a protein. After protein binding, PMBN (MPC polymer) encapsulating verteporfin is intravenously administered and is then accumulated in a cancerous area through an antigen-antibody reaction or a ligand receptor reaction. Therefore, since the maximum effect can be expected with a lower dose, the polymer is thought to have an effect of alleviating or shortening sunlight hypersensitivity. Also, verteporfin has an absorption wavelength that is as long as 690 nm. Hence, the application of verteporfin to cancer at a deeper site or advanced cancer such as advanced gastric cancer, advanced large bowel cancer, advanced lung cancer, brain tumor, and the like can be expected. Moreover, PMBN (MPC polymer) to which a cancer antigen-specific antibody has bound can also selectively reach a cancerous area, so that the dose of PMBN may be low and the expression of an anaphylactic reaction can be suppressed.

PDT using verteporfin PMBN (MPC polymer) bound to a protein causes fewer hypofunctions in tissue after treatment and is minimally invasive. Also, in the case of PDT, a brief course of therapy is sufficient for an adverse reaction after therapy. Therefore, PDT can be expected to serve as a form of therapy that can increase patients' QOL (quality of life).

The present invention is as follows.
[1] A cancer-cell-specific polymer compound, containing a photosensitizer for photodynamic therapy and having a surface to which an antibody against a cancer-cell-specific antigen binds.
[2] The cancer-cell-specific polymer compound according to [1], wherein the polymer is a copolymer having a constitutional unit based on MPC and a constitutional unit based on a hydrophobic monomer.
[3] The cancer-cell-specific polymer compound according to [2], wherein the polymer is a copolymer of MPC and n-butyl methacrylate.
[4] The cancer-cell-specific polymer compound according to [1], wherein the polymer is a liposome or a liposome-like macromolecular aggregate.
[5] The cancer-cell-specific polymer compound according to any one of [1] to [4], which contains a monomer compound containing an active ester group.
[6] The cancer-cell-specific polymer compound according to [5], wherein the monomer compound containing an active ester group is p-nitrophenyloxycarbonyl poly(ethylene glycol) methacrylate.
[7] The cancer-cell-specific polymer compound according to any one of [1] to [6], wherein the antibody against a cancer-cell-specific antigen is selected from the group consisting of an anti-EGFR antibody, an anti-EpCAM antibody, and an anti-c-erbB-2 antibody.
[8] The cancer-cell-specific polymer compound according to any one of [1] to [7], wherein the antibody against a cancer-cell-specific antigen binds to a monomer compound via an active ester group of the monomer compound containing the active ester group.
[9] The cancer-cell-specific polymer compound according .to any one of [1] to [8], wherein the photosensitizer for photodynamic therapy is verteporfin.
[10] A photodynamic therapeutic agent, containing the cancer-cell-specific polymer compound according to any one of [1] to [9].
[11] The photodynamic therapeutic agent according to [10], which is an intravenous injection preparation.
[12] A method for producing a photodynamic therapeutic agent, comprising mixing MPC, n-butyl methacrylate, and a monomer compound containing an active ester group, producing PMBN (MPC polymer), mixing the resultant with an antibody against a cancer-cell-specific antigen, causing the antibody to bind to the active ester group of p-nitrophenyloxycarbonyl poly(ethylene glycol) methacrylate , mixing the resultant with a photosensitizer for photodynamic therapy, and then causing PMBN (MPC polymer) to encapsulate the photosensitizer for photodynamic therapy.
[13] The method for producing a photodynamic therapeutic agent according to [12], wherein the monomer compound containing an active ester group is p-nitrophenyloxycarbonyl poly(ethylene glycol) methacrylate.
[14] The method for producing a photodynamic therapeutic agent according to [12] or
[13], wherein the antibody against a cancer-cell-specific antigen is selected from the group consisting of an anti-EGFR antibody, an anti-EpCAM antibody, and an anti-c-erbB-2 antibody.
[15] The method for producing a photodynamic therapeutic agent according to any one of [12] to [14], wherein the photosensitizer for photodynamic therapy is verteporfin.

This description includes the disclosure of the description and drawings of Japanese Patent Application No. 2008-321500, from which the present application claims priority.

### Brief Description of the Drawings

Fig. 1 shows the tissue disposition of verteporfin-PMBN (MPC polymer) as examined using an epidermoid cancer model. Fig. 1A shows the results for PMBN (MPC polymer) to which an anti-EGFR antibody bound. Fig. 1B shows the results for the MPC polymer to which no anti-EGFR antibody bound.
Fig. 2 shows the results for verteporfin-PMBN (MPC polymer)-laser therapy as examined using a human epidermoid cancer model.
Fig. 3 shows the results of a verteporfin PMBN (MPC polymer) incorporation test using cultured cells.
Fig. 4 shows the results of a laser therapy using verteporfin-PMBN (MPC polymer) to which an antibody bound or no antibody bound and a human large bowel cancer model.
Fig. 5 shows the results (mouse survival%) of verteporfin-PMBN (MPC polymer)·laser therapy as examined using a human epidermoid cancer model.
Fig. 6 shows the incorporation of Visudyne, a verteporfin-containing polymer (without antibody) (Ab(-) verteporfin-PMBN), and a verteporfin-containing polymer (with antibody) (Ab(+) verteporfin-PMBN) into skin after 1 hour.
Fig. 7 shows the accumulation of Visudyne and that of a verteporfin-containing polymer (with antibody) (Ab(+) verteporfin-PMBN) in tumors.

### Best Modes for Carrying out the Invention

Hereafter, the present invention is described in detail.

In the present invention, a liposome or a polymer forming a liposome-like aggregate can be used.

The term "liposome" generally refers to a closed vesicle having a lipid layer formed by clustering of phospholipids, glucolipids, and the like in a membranous form. As a liposome of the present invention, a known liposome can be used. Examples of lipids composed of the liposome of the present invention include phosphatidylcholines, phosphatidylethanolamines, phosphatidic acids, long chain alkyl phosphates, gangliosides, glucolipids, phosphatidylglycerols, and cholesterols. Examples of phosphatidycholines include dimyristoyl phosphatidylcholine, dipalmitoyl phosphatidylcholine, and distearoyl phosphatidylcholine. Examples of phosphatidylethanolamines include dimyristoyl phosphatidylethanolamine, dipalmitoyl phosphatidylethanolamine, and distearoyl phosphatidylethanolamine. Examples of phosphatidic acids and long chain alkyl phosphates include dimyristoylphosphatidic acid, dipalmitoylphosphatidic acid, distearoylphosphatidic acid, and dicetyl phosphate. Examples of gangliosides include ganglioside GM1, ganglioside GD1a, and ganglioside GT1b. Examples of glucolipids include galactosylceramide, glucosylceramide, lactosylceramide, phosphatid, and globoside. Examples of phosphatidylglycerols include dimyristoyl phosphatidylglycerol, dipalmitoyl phosphatidylglycerol, and distearoyl phosphatidylglycerol. Of these examples, phosphatidic acids, long chain alkyl phosphates, gangliosides, glucolipids, and cholesterols have the effect of increasing the stability of liposomes. Liposomes can be produced by a known method such as a thinfilm method, an antiphase evaporation method, an ethanol injection method, a dehydration-rehydration method, an ultrasonic irradiation method, an extrusion method, a French press method, or a homogenization method. Of these examples, the particle size of liposomes can be regulated using an ultrasonic irradiation method, an extrusion method, a French press method, a homogenization method, or the like. The average particle size of the liposome of the present invention ranges from 30 nm to 500 nm, preferably ranges from 50 nm to 300 nm, and further preferably ranges from 70 nm to 150 nm.

As described above, a polymer having a constitutional unit based on MPC and a constitutional unit based on a hydrophobic monomer is referred to as PMBN or an MPC polymer. The polymer having a hydrophobic unit and a hydrophilic unit is characterized in that it can maintain water solubility because of its hydrophilic unit while binding to a hydrophobic substance such as verteporfin because of its hydrophobic unit. PMBN (MPC polymer) has a constitutional unit based on MPC and a constitutional unit based on a hydrophobic monomer, wherein the constitutional molar ratio of MPC to hydrophobic monomer ranges from 20:80 to 95:5 and preferably ranges from 30:70 to 80:20. PMBN (MPC polymer) is as described in JP Patent Publication (Kokai) No. 2004-175752 A, based on which PMBN can be produced.

MPC is represented by the following formula (1).

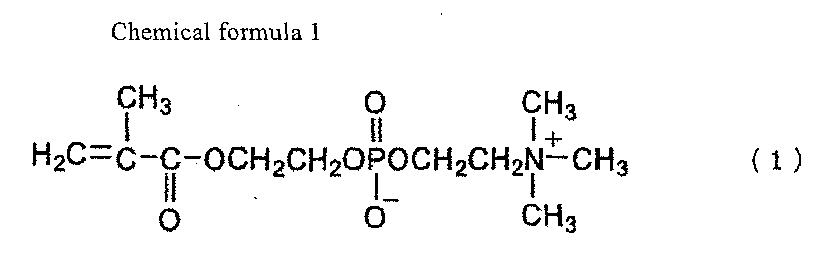

Examples of a hydrophobic monomer to be copolymerized with MPC include various monoalkyl(meth)acrylates such as methyl(meth)acrylate, ethyl(meth)acrylate, butyl(meth)acrylate, dodecyl(meth)acrylate, cetyl(meth)acrylate, stearyl(meth)acrylate, cyclohexyl(meth)acrylate, and 2-ethylhexyl(meth)acrylate; reactive functional group-containing (meth)acrylates such as glycidyl(meth)acrylate and (meth) acryloyloxyethyltriethoxysilane; urethane-modified (meth)acrylatedietylfumarates such as 2-(meth) acryloyloxyethylbutylurethane, 2-(meth) acryloyloxyethylbenzylurethane, and 2-(meth) acryloyloxyethylphenylurethane; and acrylonitrile. Of these examples, as a hydrophobic monomer, alkyl(meth)acrylate represented by formula (2) is preferable and n-butyl methacrylate (BMA) is more preferable. 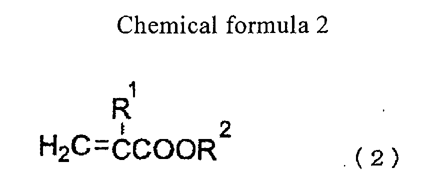 [wherein R¹ indicates H or CH₃ and R² indicates H or a C₁₋₁₈ alkyl group.]

Also, as a hydrophobic monomer to be used in the present invention, 2 or more types of the above hydrophobic monomer may be mixed and then used.

As a copolymer, any form of random copolymer or block copolymer may be used herein, and a random copolymer is preferable.

In a copolymer to be used in the present invention, the constitutional molar ratio of MPC to a hydrophobic monomer ranges from 20 : 80 to 95 : 5 and preferably ranges from 30 : 70 to 80 : 20. With less than 20 mol% MPC, the solubility in water of the resulting copolymer decreases. With more than 95 mol% MPC, the re-solubility of the composition containing the MPC polymer becomes poor. Hence, these examples of mol% are undesirable.

The weight-average molecular weight of a copolymer to be used in the present invention preferably ranges from 1000 to 5000000 and more preferably ranges from 10000 to 500000.

The above copolymer can be prepared by radical polymerization or the like of the above monomers using a known method such as solution polymerization, bulk polymerization, emulsion polymerization, or suspension polymerization, which involves substituting a polymerization system with an inert gas, such as nitrogen or argon, as necessary, and performing radical polymerization under conditions of general polymerization temperatures ranging from 10°C to 100°C and a polymerization time period ranging from 10 minutes to 48 hours. Upon polymerization, a polymerization initiator can be used. As such a polymerization initiator, 2,2'-azobis(2-amidinopropane) dihydrochloride, 4,4'-azobis(4-cyanovaleric acid), 2,2'-azobis(2-(5-methyl-2-imidazolin-2-yl)propane) dihydrochloride, 2,2'-azobis(2-(2-imidazolin-2-yl)propane) dihydrochloride, 2,2'-azobisisobutylamide dihydrate, ammonium persulfate, potassium persulfate, benzoyl peroxide, diisopropylperoxydicarbonate, t-butyl peroxy-2-ethylhexanoate, t-butyl peroxy pivalate, t-butyl peroxy diisobutyrate, lauroyl peroxide, azobisisobutyronitrile, 2,2'-azobis(2,4-dimethylvaleronitrile), or t-butyl peroxyneodecanoate (trade name "PERBUTYL ND," (NOF Corporation)), a mixture thereof, or the like can be used. For the above polymerization initiators, various redox-based accelerators may also be used. The amount of a polymerization initiator to be used herein ranges from preferably 0.01 to 20 parts by weight with respect to 100 parts by weight of a monomer composition. The thus generated copolymer can be purified by a general purification method such as a re-precipitation method, a dialysis method, and an ultrafiltration method.

Moreover, the polymer of the present invention is required to have, on its surface, a functional group for binding of an antibody against a cancer-cell-specific antigen. An example of such a functional group is an active ester group. The term "active ester group" refers to an ester group having an electron-accepting group with high acidity as one substituent of the ester and being activated against a nucleophilic reaction. Examples of an active ester group include a p-nitrophenyl active ester group, an N-hydroxysuccinimide active ester group, a phthalic acid imide active ester group, and a 5-norbornene-2,3-dicarboximide active ester group. Of these examples, a p-nitrophenyl active ester group or an N-hydroxysuccinimide active ester group is preferable and a p-nitrophenyl active ester group is particularly preferable. When the polymer of the present invention is produced, a monomer compound containing an active ester group is mixed during a polymerization reaction and then copolymerization is performed.

An example of a compound containing an active ester group is a constitutional unit (p-nitrophenyl oxycarbonyloxy poly(ethylene glychol) methacrylate (MEONP))-derived polymer represented by the following formula (3): 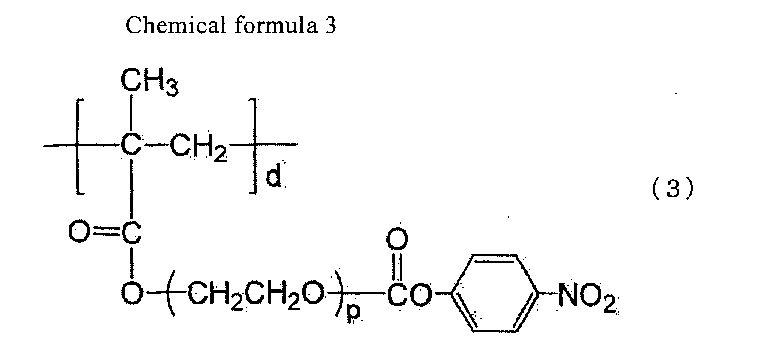 [wherein "d" ranges from 0.01 to 0.30 (preferably ranges from 0.01 to 0.20) and "p" indicates an integer between 1 and 10.]
When the polymer of the present invention is produced, the copolymer represented by formula (1) or (2) above is subjected to polymerization so that it further contains the monomer constitutional unit represented by formula (3) above and thus the copolymer can be produced. In the above formula, the value for "d" indicates the ratio of the number of the constitutional unit represented by formula (3) above to the total number of monomer constitutional units composing the copolymer.

Types of cancer for which a cancer-cell-specific antigen is expressed are not limited and examples thereof include brain-nerve tumor, skin cancer, gastric cancer, lung cancer, liver cancer, lymphoma-leukemia, cancer of the colon, pancreatic cancer, anus-rectal cancer, esophageal cancer, uterine cancer, mammary cancer, adrenal cancer, renal cancer, renal pelvic and ureteral cancer, bladder cancer, prostate cancer, urethral cancer, penile cancer, testicular cancer, bone-osteosarcoma, leiomyoma, rhabdomyoma, and mesothelioma.

Examples of tumor cell-specific antigens to be expressed on the surfaces of cancer cells include EGFR (epithelial cell growth factor), EpCAM, c-erbB-2, CCR5, CD19, HER-3, HER-4, PSMA, CEA, βhCG, CD20, CD33, CD30, CCR8, TNF-α precursor, STEAP, Mesothelin, A33 antigen, prostate stem cell antigen (PSCA), Ly-6, and CD63. Of these examples, EGFR, EpCAM, and c-erbB-2 are preferable. EGFR is expressed on squamous cell carcinoma cells, EpCAM is expressed on various cancer cells, and c-erbB-2 is expressed on cells of adenocarcinoma.

An antibody against a cancer-cell-specific antigen binds to the surface of the polymer of the present invention, and thus such antibody is directed toward cancer cells via active targeting. Specifically, the polymer of the present invention has cell-specific active accumulation properties.

An antibody can be prepared by a known method and is preferably a monoclonal antibody. An antibody may bind to PMBN (MPC polymer) via covalent bonding between an active ester group of a polymer and an amino group of amino acids composing the antibody. An antibody may also bind to a polymer by mixing a polymer and an antibody in a buffer such as a phosphate buffer. The reaction may be performed at 3°C to 37°C for 1 hour to 24 hours, for example. The mixing ratio of a polymer and an antibody is not limited. For example, 0.1 mg to 5 mg of an antibody may be mixed with 10 mg of a polymer. The amount of a binding antibody can be determined as follows. Upon antibody binding, p-nitrophenol to be released as a result of a reaction with an amino group of an antibody has an ultraviolet ray absorption peak of 400 nm. Such absorption is measured so that an approximate amount (of binding antibodies) can be quantitated. Also, after separation of unreacted antibodies by dialysis or the like, the number of antibodies is directly determined, and thus the amount of binding antibodies can be found. Moreover, antibodies, including unreacted antibodies, are subjected to separation by liquid chromatography, so that the approximate amount (of binding antibodies) can be determined.

Furthermore, the polymer of the present invention contains a fat-soluble (hydrophobic) photosensitizer for PDT. A fat-soluble photosensitizer binds to a hydrophobic portion of the polymer. A fat-soluble photosensitizer binds to a hydrophobic portion of the polymer, so that the polymer is a particulate substance encapsulating the fat-soluble photosensitizer.

Examples of a fat-soluble photosensitizer for PDT include porphyrins such as protoporphyrin and benzoporphyrin, and verteporfin. Also, these agents may be mixed and then used. Of these examples, verteporfin is preferable. In the present invention, PMBN (MPC polymer) containing verteporfin and having a surface to which an antibody binds is referred to as "Ab(+) verteporfin-PMBN (MPC polymer)," for example.

The present invention encompasses a pharmaceutical composition for cancer therapy that is a photodynamic therapeutic agent containing a cancer-cell-specific polymer compound that is a tumor cell-specific polymer compound containing a photosensitizer for photodynamic therapy and having a surface to which an antibody against a cancer-cell-specific antigen binds, wherein the polymer is a copolymer having a constitutional unit based on MPC and a constitutional unit based on a hydrophobic monomer. The pharmaceutical composition is dissolved in an appropriate buffer such as saline or phosphate buffered saline and then a pharmaceutically acceptable additive is added, as necessary. Examples of an additive include a solubilizing agent such as an organic solvent, a pH adjusting agent such as acid or base, a stabilizer such as ascorbic acid, an excipient such as glucose, and a tonicity agent such as sodium chloride.

The pharmaceutical composition of the present invention may be systemically administered in advance to a subject to be treated via intravenous injection, or it may be locally administered to a specific cancer tissue site. The dose of the pharmaceutical composition is not limited. For example, several µl to several ml of the pharmaceutical composition prepared so as to contain a photosensitizer for photodynamic therapy at several µg/ml to several mg/ml may be administered, or it may be administered directly to a target site via injection or the like. The method of administration is not limited. The pharmaceutical composition may be administered by intravenous injection, intramuscular injection, subcutaneous injection, oral administration, or the like. Of these examples, intravenous injection is preferable. Even when the photodynamic therapeutic agent of the present invention is administered by intravenous injection, it reaches cancer tissue via active targeting and can exhibit therapeutic effects against cancer. Specifically, the photodynamic therapeutic agent of present invention is preferably an intravenous injection preparation.

When the pharmaceutical composition of the present invention is administered, an antibody (against a cancer-cell-specific antigen) of the polymer is directed toward cancer cells, binds to the cancer cells (or is incorporated into the cancer cells in some cases), and then releases the photosensitizer in cancer cells. Thus the photosensitizer is accumulated within cancer cells. Also, in some cases, the photosensitizer can be released around the cancer cells, allowing the photosensitizer to accumulate around the cancer cells.

At 0.5 hours to 72 hours after administration of the pharmaceutical composition, a subject is irradiated with light and then photodynamic therapy is performed. Irradiation light can be appropriately determined depending on the photosensitizer used. Specifically, light having the same wavelength as the absorption wavelength of a photosensitizer may be used for irradiation. For example, when the photosensitizer is verteporfin, light having a wavelength of 690 nm may be administered. When the photosensitizer is Photofrin, light having a wavelength of 630 nm may be administered. Light to be administered may be light emitted from a semiconductor laser or a dye laser, for example. Specifically, an Nd-YAG (yttrium-aluminium-garnet) laser, a CO₂ laser, an argon dye laser, an excimer laser, or the like can be used. A subject may be subjected to whole-body irradiation with light. Alternatively, a catheter containing optical fiber is inserted *in vivo* into a blood vessel or the like and then an affected tumor site may be topically irradiated with light via the optical fiber.

The photodynamic therapeutic agent of the present invention is useful for additional therapy in cases of positive margin after demucosation (that is, cases in which cancer remains beneath the mucous membrane after excision of a cancerous area from the mucous membrane (early cancer of the mucous membrane) via endoscopic operation), for example.

Furthermore, after administration of the pharmaceutical composition of the present invention, ultrasonic irradiation may be performed instead of light irradiation. A photosensitizer such as verteporfin is irradiated with ultrasonic waves, so that free radicals are generated and the growth of cancer cells can be suppressed. Ultrasonic waves reach deep sites of the body and thus are useful for the treatment of cancer in areas deep within the body. For example, the pharmaceutical composition of the present invention is administered *in vivo* and then the affected tumor site may be irradiated with ultrasonic waves. Ultrasonic intensity ranges from 0.1 W/cm² to 10 W/cm² and preferably ranges from 1 W/cm² to 5 W/cm^{2,} and the time period for ultrasonic irradiation ranges from several seconds to several hundred seconds, but examples are not limited thereto.

The present invention will be described in more detail below with reference to examples. However, the present invention is not limited to such examples.

### Example 1 Preparation of verteporfin-encapsulated PMBN (MPC polymer)

### Synthesis of p-nitrophenyloxycarbonyl ethyleneglycol methacrylate (MEONP)

Poly(ethylene glycol) monomethacrylate (0.01 mol) was dissolved in 20 mL of chloroform and then cooled to -30°C. The solution was kept at -30°C. A homogenous solution prepared in advance containing 0.01 mol of p-nitrophenyl chloroformate, 0.01 mol of triethylamine, and 20 mL of chloroform were added dropwise. A reaction was performed at -30°C for 1 hour, following which the solution was further agitated at room temperature for 2 hours. Subsequently, salt was removed by filtration from the reaction solution and then the solvent was distilled off, so that p-nitrophenyloxycarbonyl poly(ethylene glycol) methacrylate (MEONP) was obtained.

### Preparation of MPC polymer

MPC, hydrophobic n-butyl methacrylate (MBA), and p-nitrophenyloxycarbonyl poly(ethylene glycol) methacrylate (MEONP) having an active ester were mixed at a molar ratio of 60:20:20. Moreover, AIBN was used as a polymerization initiator, following which the solution was diluted with ethanol. After substitution of the reaction system with argon, a reaction was performed at 60°C for 5 hours, and thus a copolymer was prepared. For polymer purification, a re-precipitation method and a membrane dialysis method were employed.

The thus obtained polymer (10 mg) was dissolved in PBS supplemented with 1 mg of an anti-EGFR antibody (528; ATCC accession number: HB8509) to a final volume of 5 ml, followed by overnight reaction at 4°C. Subsequently; 100 µl of 20 mg/ml verteporfin was added. Thus, PMBN (MPC polymer) containing verteporfin encapsulated therein and having a surface to which the anti-EGFR antibody had bound was obtained.

### Example 2 Examination of tissue disposition of verteporfin-PMBN (MPC polymer) using epidermoid cancer model

A431 human epidermoid cancer cells (1 × 10⁷ cells) were subcutaneously administered to 6-week-old BALB/c nude mice. After the subcutaneous tumor had grown to about 5 mm in diameter, 300 µl of PMBN (MPC polymer) containing verteporfin prepared in Example 1 was intravenously injected, following which the tissue was collected. The thus collected tissue was freeze-dried and then the dry weight was measured. N,N-dimethylformamide was added to the freeze-dried tissue and the resultant was then homogenized. After elution of verteporfin, absorbance was measured at OD686 nm and then incorporation amounts were calculated. At this time, an MPC polymer to which the anti-EGFR antibody had bound and PMBN (MPC polymer) to which no anti-EGFR antibody had bound were used.

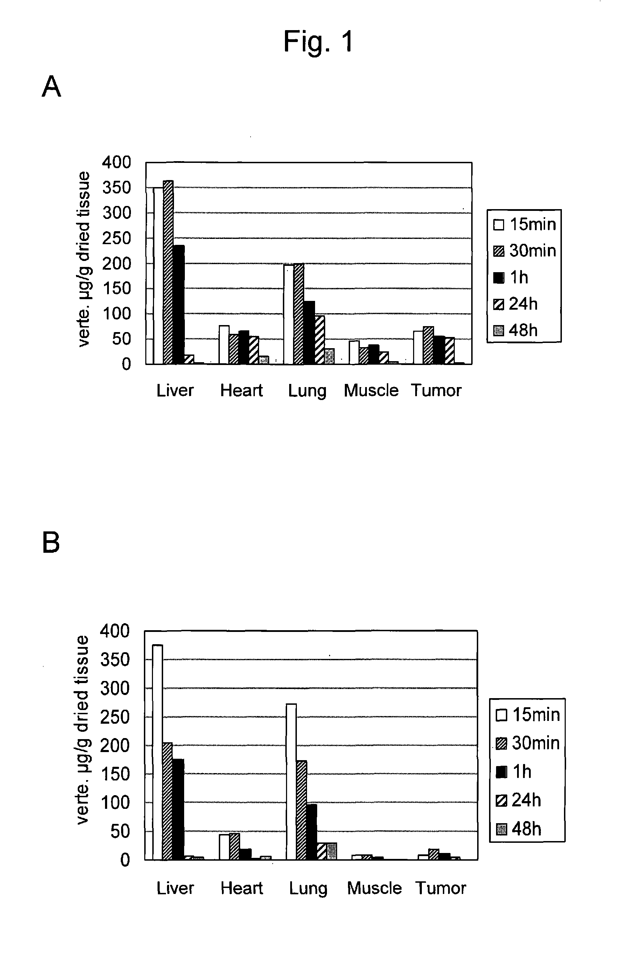

Fig. 1 shows the results. Fig. 1A shows the results for PMBN (MPC polymer) to which the anti-EGFR antibody had bound. Fig. 1B shows the results for PMBN (MPC polymer) to which no anti-EGFR antibody had bound. As shown in Fig. 1, the MPC polymer to which no anti-EGFR antibody had bound was almost never incorporated by the tumor tissue, but the MPC polymer to which the anti-EGFR antibody had bound was incorporated in large amounts into the tumor tissue.

### Example 3 Examination of verteporfin-PMBN (MPC polymer)-laser therapy using human epidermoid cancer model

A431 human epidermoid cancer cells (1 × 10⁷) were subcutaneously administered to 6-week-old BALB/c nude mice. After the subcutaneous tumor had grown to about 5 mm, 300 µl of verteporfin-PMBN (MPC polymer) prepared in Example 1 was intravenously injected. At 1 hour after intravenous injection, laser irradiation (75 J/cm²) was performed using a 640-nm Fiber Coupled LD model (SONY) and then the tumor size was measured. At this time, an MPC polymer to which the anti-EGFR antibody had bound and an MPC polymer to which no anti-EGFR antibody had bound were used.

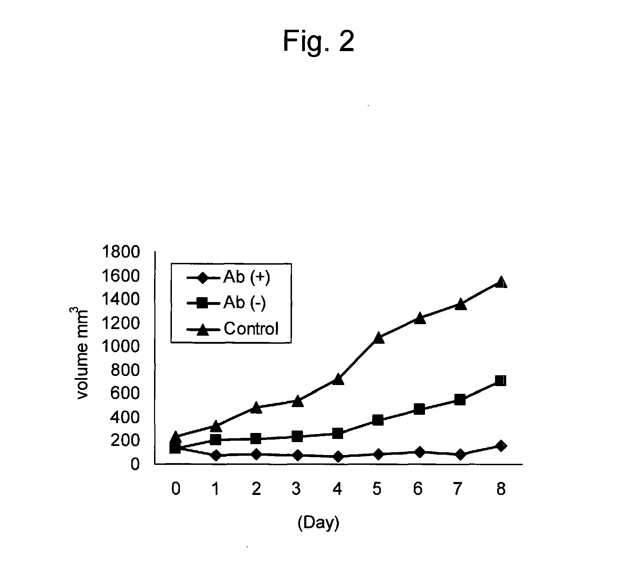

Fig. 2 shows the results. In Fig. 2, "control" indicates a nude mouse to which no PMBN (MPC polymer) had been administered. As shown in Fig. 2, tumor volume was found to increase over time in the case of the control. In the case of administration of PMBN (MPC polymer) to which no anti-EGFR antibody had bound, the degree of increase in tumor volume was lower than that of the control, but tumor volume was found to increase to some extent. In the case of administration of the MPC polymer to which the anti-EGFR antibody had bound, almost no increase in tumor volume was observed.

### Observation of incorporation of verteporfin

A431 was seeded on a 24-well culture dish and then the dish was left to stand for about 24 hours until adhesion was completed.

Verteporfin PMBN (MPC polymer) (100 µl) with or without antibody was added to 1 ml of a medium. After 3 instances of washing with PBS at 10 minutes, 20 minutes, and 30 minutes after addition, respectively, new medium was added. One hour later, observation was performed using a Leica TCS-SP5 confocal laser microscope at excitation of 488 nm and 650 nm-700 nm fluorescence.

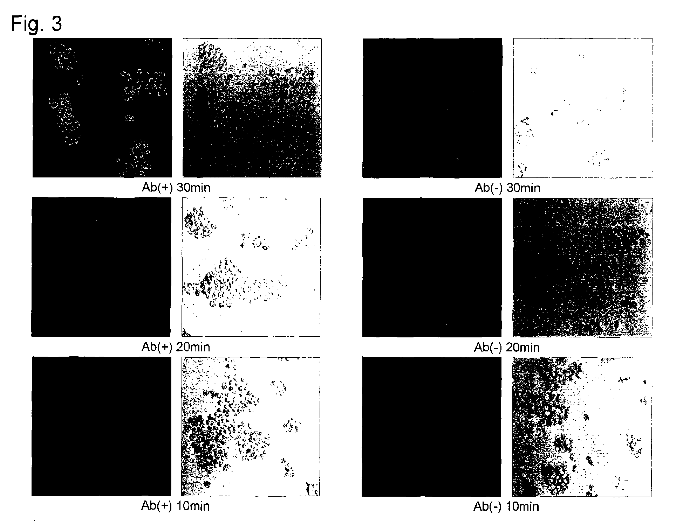

Fig. 3 shows fluorescent images for observation. In Fig. 3, "Ab(+)" indicates the results when an MPC polymer to which anti-EGFR antibody had bound was administered. "Ab(-)" indicates the results when an MPC polymer to which no anti-EGFR antibody had bound was administered. Each fluorescent image shows A431 cells that had been in contact with verteporfin MPC polymer for 10 minutes, 20 minutes, or 30 minutes, respectively. As shown in Fig. 3, when PMBN (MPC polymer) to which the anti-EGFR antibody had bound was used, cells exhibited strong fluorescence as a result of staining and verteporfin was incorporated in large amounts.

### Comparative example 1 Examination of verteporfin PMBN (MPC polymer)-laser therapy using WiDr human large bowel cancer cells

Examination was conducted using a WiDr human large bowel cancer cell line expressing EGFR at a low level.

WiDr human large bowel cancer cells (1 × 10⁷ cells) were subcutaneously injected to 6-week-old BALB/c nude mice.

When the subcutaneous tumor had grown to about 5 mm, 300 µl of verteporfin MPC polymer prepared in Example 1 was intravenously injected. At this time, PMBN (MPC polymer) to which the anti-EGFR antibody had bound and an MPC polymer to which no anti-EGFR antibody had bound were used.

At 1 hour after intravenous injection, laser irradiation (75J/cm²) was performed using a 640 nm Fiber Coupled LD model (SONY) and then the tumor size was measured.

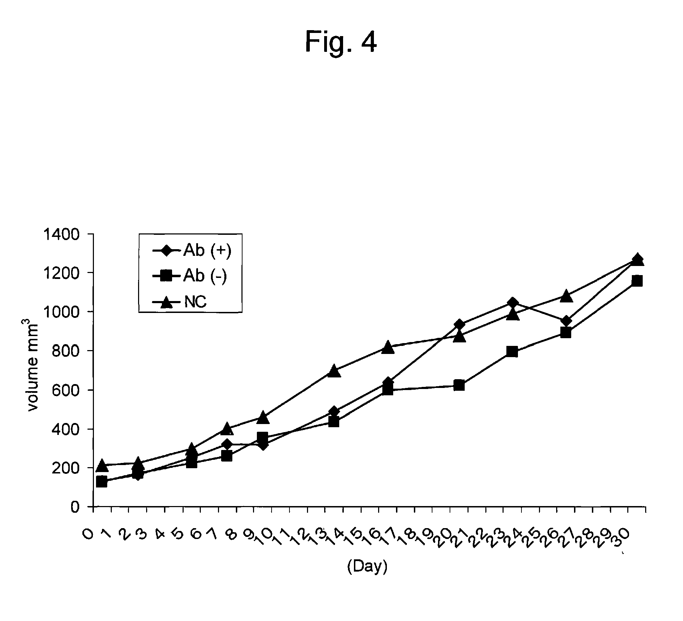

Fig. 4 shows the results when the MPC polymer to which the anti-EGFR antibody had bound or no anti-EGFR antibody had bound was administered.

As shown in Fig. 4, when the WiDr human large bowel cancer cell line expressing EGFR at a low level was used, the therapeutic effects of the MPC polymer to which the anti-EGFR antibody had bound was not observed.

### Example 4 Examination (examination of survival rate) of verteporfin-PMBN (MPC polymer)-laser therapy using human epidermoid cancer model

A431 human epidermoid cancer cells (1 × 10⁷ cells) were subcutaneously administered to 6-week-old BALB/c nude mice. After the subcutaneous tumor had grown to about 5 mm, 300 µl of verteporfin-PMBN (MPC polymer) prepared in Example 1 was intravenously injected. At 1 hour after intravenous injection, laser irradiation (75J/cm²) was performed using a 640-nm Fiber Coupled LD model (SONY) and then mouse survival rate was monitored over time. At such time, an MPC polymer to which the anti-EGFR antibody had bound and PMBN (MPC polymer) to which no anti-EGFR antibody had bound were used.

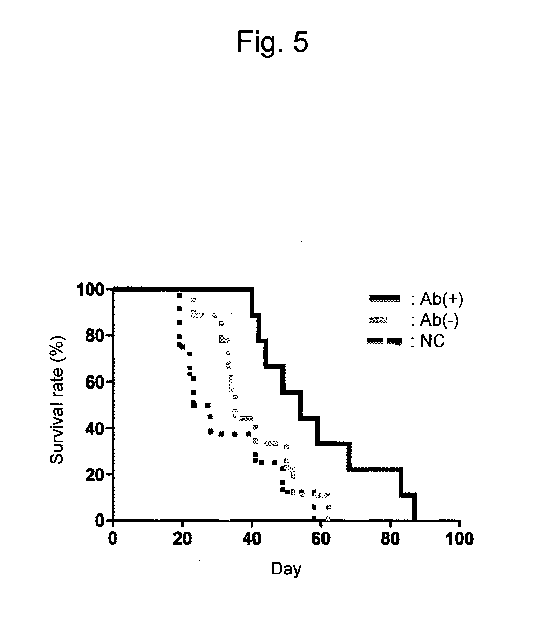

Fig. 5 shows mouse survival rate results. In Fig. 5, "Ab(+)" and "Ab(-)" indicate the results for PMBN (MPC polymer) to which the anti-EGFR antibody bound and the polymer to which no anti-EGFR antibody bound, respectively. Also, NC indicates the results for a control to which no PMBN (MPC polymer) was administered. As shown in Fig. 5, the survival rate was improved in mice to which PMBN (MPC polymer to which the anti-EGFR antibody had bound) had been administered.

### Example 5 Comparison of the effects between antibody-bound PMBN (MPC polymer) and Visudyne

The tissue disposition of PMBN (MPC polymer) was examined by the following method.

A431 human epidermoid cancer cells (1 × 10⁷ cells) were subcutaneously administered to 6-week-old BALB/c nude mice. After the subcutaneous tumor had grown to about 5 mm in diameter, 300 µl of PMBN (MPC polymer) containing verteporfin prepared in Example 1 was intravenously injected and then the tissue was collected. The weight of the collected tissue was measured and then freeze-dried. N,N-dimethylformamide was added to the tissue, the tissue was homogenized. After elution of verteporfin, absorbance was measured at OD686 nm and then the incorporation amount was calculated.

Agents used herein were Visudyne, a polymer (without antibody) containing verteporfin (Ab(-) verteporfin-PMBN), and a polymer (with antibody) containing verteporfin (Ab(+) verteporfin-PMBN). Incorporation of these agents into normal tissues (skin and muscle) and accumulation of the same in tumor were examined. Visudyne is a remedy for age-related macular degeneration, and it is prepared by adding the following ingredients (contents per vial: 15 mg of verteporfin; 690 mg of lactose as an additive; 48.75 mg of egg phosphatidylglycerol; 70.50 mg of dimyristoylphosphatidylcholine; 0.15 mg of palmitic acidascorbic acid; and 0.015 mg of dibutyl hydroxytoluene) for solubilization of hydrophobic verteporfin. In addition, in an experiment comparing the effects of Visudyne, a polymer containing verteporfin (without antibody) (Ab(-) verteporfin-PMBN), and a polymer containing verteporfin (with antibody) (Ab(+) verteporfin-PMBN), the same amounts of verteporfin were used.

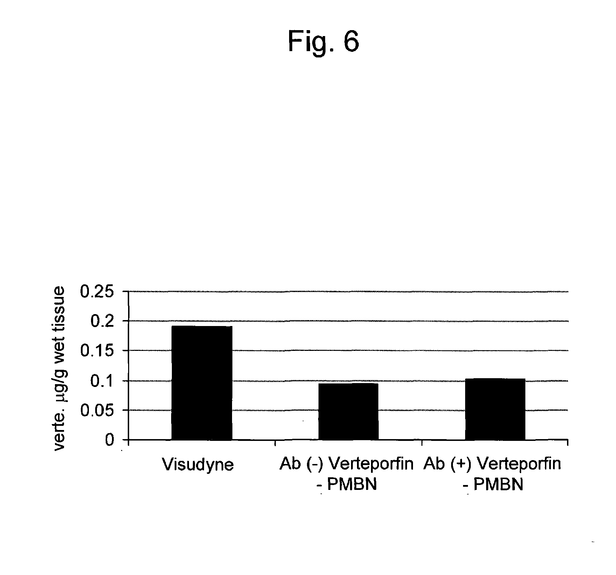

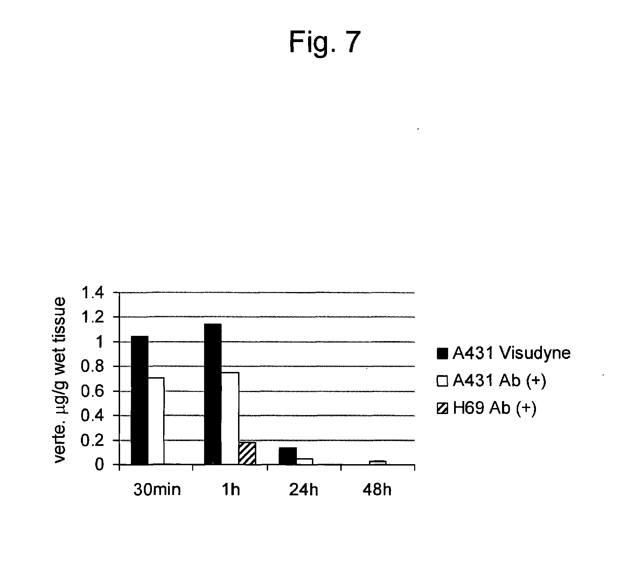

Fig. 6 and Fig. 7 show the results. Fig. 6 shows incorporation into the skin after 1 hour and Fig. 7 shows accumulation in a tumor over time.

As shown in Fig. 6, when verteporfin was administered in the same dose, regardless of the presence or the absence of the antibody, incorporation into the skin was inhibited to a level half of that in cases in which Visudyne was used because of the use of the polymer containing verteporfin. As a result, the incidence of photosensitivity can be reduced. This may be due to the fact that the particle size of the polymer increased to 40 nm to 60 nm (without antibody) and to 100 nm to 120 nm (with antibody), compared with the case of Visudyne.

Also, as understood from Fig. 7 showing the results of accumulation in a tumor, Visudyne and the polymer containing verteporfin (with antibody) (Ab(+) verteporfin-PMBN) were highly incorporated into A431 cells expressing EGFR at high levels, but the polymer containing verteporfin (with antibody) (Ab(+) verteporfin-PMBN) was almost never incorporated into EGFR-negative H69 cells. The results suggest that incorporation into and accumulation in a tumor do not depend on the Enhanced Permeability and Retention effect (EPR; whereby particles having specific sizes pass through gaps between blood vessel walls and are then accumulated in a tumor), but rather depend on the antibody.

The ratios of incorporation in a tumor (A431) to incorporation in normal tissue were calculated. Whereas the ratio of incorporation in a tumor/incorporation in skin was 4.24 (4.24:1) 30 minutes after administration of Visudyne, the same was 50.00 (50.00:1) 30 minutes after administration of Ab(+) verteporfin-PMBN; and whereas the same was 5.96 (5.96:1) 1 hour after administration of Visudyne, the same as high as 7.26 (7.26:1) 1 hour after administration ofAb(+) verteporfin-PMBN.

### Industrial Applicability

The photodynamic therapeutic agent of the present invention encapsulates a photosensitizer and has a surface to which an antibody (specific to an antigen that is expressed specifically on the surfaces of cancer cells) binds. Accordingly, when systemic medication of the photodynamic therapeutic agent is performed via intravenous injection, active targeting enables a photosensitizer to efficiently reach the cancer tissue with a smaller dose of agent. When the photodynamic therapeutic agent of the present invention is used for cancer therapy, complications due to sun sensitivity are unlikely to occur. Also, when verteporfin is used as a photosensitizer, the dose can be lowered and *in vivo* concentration can be kept at a low level, and thus the fear of inducing an anaphylactic reaction will decrease.

All publications, patents, and patent applications cited in this description are herein incorporated by reference in their entirety.

## Claims

1. A cancer-cell-specific polymer compound, containing a photosensitizer for photodynamic therapy and having a surface to which an antibody against a cancer-cell-specific antigen binds.

2. The cancer-cell-specific polymer compound according to claim 1, wherein the polymer is a copolymer having a constitutional unit based on 2-methacryloyloxyethyl phosphorylcholine (hereinafter, abbreviated as MPC) and a constitutional unit based on a hydrophobic monomer.

3. The cancer-cell-specific polymer compound according to claim 2, wherein the polymer is a copolymer of MPC and n-butyl methacrylate.

4. The cancer-cell-specific polymer compound according to claim 1, wherein the polymer is a liposome or a liposome-like macromolecular aggregate.

5. The cancer-cell-specific polymer compound according to any one of claims 1 to 4, which contains a monomer compound containing an active ester group.

6. The cancer-cell-specific polymer compound according to claim 5, wherein the monomer compound containing an active ester group is p-nitrophenyloxycarbonyl poly(ethylene glycol) methacrylate.

7. The cancer-cell-specific polymer compound according to any one of claims 1 to 6, wherein the antibody against a cancer-cell-specific antigen is selected from the group consisting of an anti-EGFR antibody, an anti-EpCAM antibody, and an anti-c-erbB-2 antibody.

8. The cancer-cell-specific polymer compound according to any one of claims 1 to 7, wherein the antibody against a cancer-cell-specific antigen binds to a monomer compound via an active ester group of the monomer compound containing the active ester group.

9. The cancer-cell-specific polymer compound according to any one of claims 1 to 8, wherein the photosensitizer for photodynamic therapy is verteporfin.

10. A photodynamic therapeutic agent, containing the cancer-cell-specific polymer compound according to any one of claims 1 to 9.

11. The photodynamic therapeutic agent according to claim 10, which is an intravenous injection preparation.

12. A method for producing a photodynamic therapeutic agent, comprising mixing MPC, n-butyl methacrylate, and a monomer compound containing an active ester group, producing an MPC polymer, mixing the resultant with an antibody against a cancer-cell-specific antigen, causing the antibody to bind to the active ester group of p-nitrophenyloxycarbonyl poly(ethylene glycol) methacrylate, mixing the resultant with a photosensitizer for photodynamic therapy, and then causing the MPC polymer to encapsulate the photosensitizer for photodynamic therapy.

13. The method for producing a photodynamic therapeutic agent according to claim 12, wherein the monomer compound containing an active ester group is p-nitrophenyloxycarbonyl poly(ethylene glycol) methacrylate.

14. The method for producing a photodynamic therapeutic agent according to claim 12 or 13, wherein the antibody against a cancer-cell-specific antigen is selected from the group consisting of an anti-EGFR antibody, an anti-EpCAM antibody, and an anti-c-erbB-2 antibody.

15. The method for producing a photodynamic therapeutic agent according to any one of claims 12 to 14, wherein the photosensitizer for photodynamic therapy is verteporfin.
